# EUROPÄISCHE PATENTSCHRIFT

(11) **EP 1 037 589 B1**
(45) Veröffentlichungstag und Bekanntmachung des Hinweises auf die Patenterteilung: **05.03.2003**
(21) Anmeldenummer: 98962416.8
(22) Anmeldetag: 04.12.1998
(51) Int. Cl.: A61K 7/00, A61K 7/09, A61K 7/13, A61K 7/135

(54) **MITTEL ZUR BEHANDLUNG KERATINISCHER FASERN**
AGENTS FOR TREATING KERATIN FIBRES
AGENTS POUR LE TRAITEMENT DE FIBRES KERATINIQUES

(30) Priorität: 13.12.1997 DE 19755420
(43) Veröffentlichungstag der Anmeldung: 27.09.2000
(73) Patentinhaber: Hans Schwarzkopf GmbH & Co. KG, 22763 Hamburg (DE)
(72) Erfinder: BARTELS, Holger, D-22761 Hamburg (DE); WOLFF, Wolfgang, D-22951 Bargteheide (DE); HOEPFNER, Stefan, D-22763 Hamburg (DE); ROHWEDER, Sandra, D-22763 Hamburg (DE)
(74) Vertreter: Foitzik, Joachim Kurt
(86) Internationale Anmeldenummer: EP9807905
(87) Internationale Veröffentlichungsnummer: WO99030673

(56) Entgegenhaltungen:
- WO-A-91/01127
- FR-A- 2 718 352

## Beschreibung

Die Erfindung betrifft Mittel zur Behandlung keratinischer Fasern, bestehend aus mindestens zwei räumlich getrennt vorliegenden Zubereitungen, deren Verwendung sowie ein Verfahren zur Behandlung menschlicher Haare.

Die Anforderungen, die Anwender von Mitteln zur Behandlung keratinischer Fasern, insbesondere Haarbehandlungsmitteln, an diese Mittel stellen, haben sich im Laufe der Zeit kontinuierlich erhöht. Dies führte u.a. dazu, daß diese Mittel in vielen Fällen aus einer immer größer werdenden Anzahl von Komponenten bestehen, um alle vom Anwender gewünschten Wirkungen in möglichst optimaler Weise zu erzielen. Mit der Komplexität dieser Mischungen steigt aber auch die Zahl der Wirkstoffkombinationen, die zwar die gewünschten Eigenschaften entfalten, in einer einzigen Formulierung, insbesondere wenn es sich um eine wäßrige Mischung handelt, jedoch nicht mehr lagerstabil sind.

Im Bereich der Haarbehandlung sind eine Reihe von Mitteln bekannt, die in Form getrennter Zubereitungen vorliegen, die entweder unmittelbar vor dem Auftragen auf das Haar gemischt werden oder in getrennten Verfahrensschritten nacheinander auf das Haar appliziert werden: zur Vereinfachung der Handhabung für den Anwender und nicht zuletzt zur Minimierung des Verpackungsaufwandes wird bei der Entwicklung neuer Produkte, wo immer möglich, die Formulierung in Form eines Mittels angestrebt.

Sollte sich die Formulierung in getrennten Zubereitungen, die vor der Applikation auf das Haar vermischt werden müssen, absolut nicht vermeiden lassen, so sollte der Mischvorgang möglichst unkompliziert und schnell gestaltbar sein und sich, wie bereits oben gesagt, der zusätzliche Verpackungsauiwand möglichst gering halten lassen.

Aus der WO 91/01127 A1 war ein "Bleichpaket" zum Bleichen von Haaren bekannt, das ein Bleichmittel in einer Hülle mit 0,25 bis 5 mm Schichtdicke umfasst.

Es wurde nunmehr gefunden, daß diese Forderungen in hohem Maße erfüllt werden, wenn die getrennt zu lagernde Komponente bzw. die getrennt zu lagemden Komponenten mit einer Umhüllung versehen werden, die bei Vereinigung der Zubereitungen eine Vermischung der Komponenten beider Zubereitungen in einem für den Anwender tragbaren Zeitraum ermöglicht. Als vom Anwender akzeptierter Zeitraum kann in der Regel ein Intervall von bis zu 5 Minuten angesehen werden, wenn der Vermischungsvorgang beider Zubereitungen mit keinerlei weiteren, aufwendigen Prozeduren, z.B. ständigem Rühren, verbunden ist.

Ein erster Gegenstand der Erfindung ist somit ein Mittel zur Behandlung keratinischer Fasern, bestehend aus mindestens einer wäßrigen Zubereitung A und mindestens einer davon räumlich getrennt vorliegenden Zubereitung B, gekennzeichnet durch eine Umhüllung der Zubereitung B aus einem Material, das bei Zugabe der Zubereitung B zu einer Zubereitung A eine Vermischung der Komponenten beider Zubereitungen bei 38 °C innerhalb von 5 Minuten ermöglicht.

Eine Reihe von Haarbehandlungsmitteln werden in leicht erwärmter Form auf das Haar appliziert. Die Erwärmung wird in der Regel durch Zusammenführung von Komponenten erreicht, die bei der Mischung Wärme abgeben. Eine Komponente ist dabei häufig ein wasserfreies Salz, das sich in einer wäßrigen Phase unter Wärmeabgabe löst; alternativ dazu kann man sich aber auch Neutralisationswärmen, Mischungswärmen flüssiger Komponenten etc. bedienen. Die Mengenverhältnisse der zu mischenden Komponenten werden in diesen Fällen vorzugsweise so eingestellt, daß die Mischung innerhalb kürzester Zeit, d.h. wenigen Sekunden, eine Temperatur geringfügig oberhalb der Temperatur des menschlichen Körpers, d.h. ca. 38 °C, annimmt. Bei dieser Art von Mitteln ist es also ausreichend, wenn die Vermischung der Komponenten der umhüllten Zubereitung B mit den Komponenten der wäßrigen Zubereitung A innerhalb von 5 Minuten bei einer Temperatur von 38 °C erfolgt. Dabei sollten, abgesehen von gelegentlichem Schütteln der Mischung, keine weiteren Aktivitäten seitens des Anwenders notwendig sein.

Die weitaus überwiegende Anzahl der Haarbehandlungsmittel werden bei Raumtemperatur gelagert und auch mit dieser Temperatur auf das Haar appliziert. Gemäß einer bevorzugten Ausführungsform der Erfindung wird die Umhüllung der Zubereitung B daher so gewählt, daß eine Vermischung der Komponenten der Zubereitungen A und B innerhalb von maximal 5 Minuten auch bei Raumtemperatur, d.h. insbesondere einer Temperatur von 20 °C, stattfindet.

Gemäß einer bevorzugten Ausführungsform der Erfindung handelt es sich bei der Umhüllung der Komponente B um eine Kapsel.

Entsprechende Umhüllungen in Kapselform sind aus der Pharmazie bekannt. Ein Typ dieser Kapseln sind Filme in Kapselform, bei denen die zu umhüllenden Stoffe vollständig von einer Schicht des Umhüllungsmaterials eingeschlossen sind und die umhüllten Stoffe nur nach Zerstörung der Umhüllung wieder freigesetzt werden können. Hierbei kommen als Materialien für die Umhüllung bevorzugt bestimmte Cellulosederivate, Polyacrylate, Polymethacrylate, Polyvinylpyrrolidone sowie insbesondere Polyvinylalkohole in Betracht. In diesem Zusammenhang wird ausdrücklich auf die Monographie von K.H. Bauer, K.-H. Frömming und C. Führer, Pharmazeutische Technologie, Gustav Fischer Verlag, Stuttgart, Jena, Lübeck, Ulm, 4. Auflage, 1997, Seiten 315-323, Bezug genommen.

Zweiteilige Kapseln bestehen in der Regel aus zwei zylindrischen Hälften, die jeweils an einem Ende verschlossen sind. Dabei ist der Innendurchmessers der einen Hälfte, die auch als Kapselkappe bezeichnet wird, geringfügig größer als der Außendurchmesser der als Kapselboden bezeichneten anderen Hälfte, so daß die beiden Hälften unter Umhüllung der einzuschließenden Komponenten ineinandergeschoben werden können, wobei die Kapsel durch spezielle Vorrichtungen "verriegelt" wird. Dieser Typ von Kapseln wird auch als Steckkapseln bezeichnet und ist eine erfindungsgemäß bevorzugte Umhüllung. Als Kapselmaterialien kommen prinzipiell sowohl Gelatine als auch synthetische oder natürliche Polymeren in Betracht. Polyvinylalkohol hat sich als besonders geeignetes Umhüllungsmaterial erwiesen.

Gemäß einer ersten bevorzugten Ausführungsform besteht die gesamte Steckkapsel aus Polyvinylalkohol.

Es kann aber auch vorgesehen sein, daß nur ein Teil der Steckkapsel, insbesondere die Kapselkappe oder Teile der Kapselkappe, aus wasserlöslichem Material gemäß erfindungsgemäßer Definition besteht. Es ist dann möglich, beispielsweise den Kapselboden aus einem weniger wasserlöslichen Material herzustellen. Man ist dadurch flexibler in der Einstellung der gewünschten mechanischen Eigenschaften, z.B. der Steifigkeit, der Kapseln. Hinsichtlich dieser Umhüllungsform wird ebenfalls auf die oben erwähnte Monographie von K.H. Bauer, K.-H. Frömming und C. Führer, Seiten 324-335, Bezug genommen.

Die Umhüllung kann aber auch in Form eines Säckchens ausgebildet sein. Solche Säckchen werden in bekannter Weise aus Polymerfolien hergestellt, aus denen durch Schweißen oder Verkleben die einzelnen, die Inhaltsstoffe enthaltenden Säckchen hergestellt werden. Nähere Informationen zu solchen Säckchen sind beispielsweise dem Aufsatz von J. Korn, Die Neue Verpackung, 10, 1150-1155 (1962) sowie der EP 493 392 zu entnehmen, in der Säckchen aus Polyvinylalkohl zur staubfreien Abpackung von Bleichmitteln offenbart werden.

Wegen der besseren Handhabbarkeit, insbesondere bei der Zugabe der Zubereitung B zu einer in einer Flasche mit vergleichsweise engem Hals vorliegenden Zubereitung A, ist eine Umhüllung in Form einer Kapsel erfindungsgemäß bevorzugt.

Weiterhin ist bevorzugt, daß das Umhüllungsmaterial aus Substanzen besteht, die in Haarbehandlungsmitteln zusätzlich noch positive Eigenschaften entfalten. Auch in diesem Zusammenhang ist Polyvinylalkohol aufgrund seiner konditionierenden Eigenschaften ein bevorzugtes Umhüllungsmaterial.

Die Schichtdicke der Umhüllung wird der Fachmann so wählen, daß einerseits ein hermetischer Abschluß der zu umhüllenden Zubereitung gewährleistet ist, andererseits aber die Vermischung der Zubereitungen A und B nicht durch eine zu dicke Umhüllung unnötigerweise verlangsamt wird. Stärken der Umhüllung in Bereich von 10 bis 30 Mikrometern haben sich im Rahmen der erfindungsgemäßen Lehre als besonders geeignet erwiesen.

Die erfindungsgemäße Lehre ist besonders geeignet für Mittel, die in der Zubereitung B einen Bestandteil enthalten, der in der Zubereitung A nicht lagerstabil ist.

Gemäß einer ersten bevorzugten Ausführungsform ist dieser Bestandteil ein Parfümöl, eine spezielle Parfümkomponente oder eine Mischung von Parfümölen.

Bestimmte Haarbehandlungsmittel enthalten Wirkstoffe, die zur Erzielung der gewünschten Eigenschaften oder zur Formulierung des Mittels unverzichtbar sind, aber einen solchen Eigengeruch entwickeln, daß eine Parfümierung praktisch unerläßlich ist, um eine Akzeptanz beim Anwender zu erzielen. Beispiele hierfür sind insbesondere Dauerwellmittel mit einem Gehalt sowohl an schwefelorganischen Verbindungen wie Thioglykolsäure, deren Salzen und/oder Derivaten als Reduktionsmittel als auch Ammoniak oder niederen Aminen zur Einstellung des pH-Wertes. Sowohl wegen der im Mittel enthaltenen Reduktionsmittel als auch wegen der genannten Alkalisierungsmittel ist der Fachmann hinsichtlich der verwendbaren Parfümkomponenten stark eingeschränkt, wenn er die Aufgabe hat, über Monate lagerstabile Mittel zu formulieren. Andererseits sind die meisten der für solche lagerstabilen Mittel ungeeigneten Parfümkomponenten in den Mitteln für einen kurzen Zeitraum von bis zu einer Stunde, also für den Zeitraum der eigentlichen Anwendung, hinreichend stabil, um die gewünschte Duftwirkung zu entfalten.

Andere Mittel gemäß dieser Ausführungsform sind Dauerwellfixiermittel, die üblicherweise stark sauer eingestellt sind und bis zu 12 Gew.% eines Oxidationsmittels, insbesondere Wasserstoffperoxid, enthalten. Auch unter diesen Bedingungen sind viele Parfümkomponenten zwar für den Zeitraum der Anwendung auf dem Haar, nicht jedoch über Monate oder Jahre stabil. Weitere Mittel, bei denen Inkompatibilitäten zwischen Parfümkomponenten und weiteren Bestandteilen auftreten, sind beispielsweise Oxidationsfärbemittel und Blondiermittel.

Die erfindungsgemäße Lehre ist insbesondere geeignet für Parfümöle und Parfümkomponenten, die eine Aldehydfünktion und/oder eine, ggf. zu der Aldehydfunktion in Konjugation stehende, C-C-Doppelbindung aufweisen. Weitere Parfümkomponenten, die eine primäre Alkoholfünktion oder eine Esterfunktion aufweisen, stellen ebenfalls bevorzugte Verbindungen im Rahmen dieser Ausführungsform der Erfindung dar.

Weiterhin weisen Parfümkomponenten der Duftrichtung "fruchtig" in besonders vielen Fällen unbefriedigende Stabilitäten in Mittel, die starke Reduktions- bzw. Oxidationsmittel aufweisen, auf. Auch für solche Parfümkomponenten hat sich die erfindungsgemäße Lehre als außerordentlich gut geeignet erwiesen. Gleiches gilt schließlich auch für die sogenannten Sandel-Riechstoffe, sowohl natürlicher als auch insbesondere synthetischer Provenienz.

Gemäß einer zweiten bevorzugten Ausführungsform handelt es sich bei dem inkompatiblen Bestandteil um einen Stoff aus der Gruppe, die aus Vitaminen, Provitaminen und ihren Derivaten gebildet wird. Diese Gruppe umfaßt beispielsweise Panthenol und seine Derivate wie Pantothensäure, Pantothenylester und -ether wie Pantothenylethylether. Insbesondere Panthenol selbst ist in stärker alkalischen oder sauren, wäßrigen Medien sowie in Oxidationsmittel enthaltenden Zubereitungen nicht hinreichend lagerstabil. Als weitere Vitamine aus dem B-Komplex sind die Vitamine B₂, B₆, B₁₂ sowie das Nicotinsäureamid zu nennen. Retinol und andere Substanzen, die als Vitamin A bezeichnet werden, sind beispielsweise empfindlich gegen Oxidationsmittel, gleiches gilt für das Vitamin E.

Die Art des Haarbehandlungsmittels unterliegt keinen prinzipiellen Beschränkungen. Es kann sich beispielsweise um reinigende Mittel wie Shampoos, pflegende Mittel wie Haarkuren und Haarspülungen, festigende Mitteln wie Haarfestiger, Haarsprays und Fönwellen, dauerhafte Verformungsmittel wie Dauerwell- und -fixiermittel, farbverändernde Mittel wie Blondiermittel, Oxidationsfärbemittel und Tönungsmittel auf Basis direktziehender Farbstoffe, Haarwässer und Haarspitzenfluids handeln. Entsprechend können die Zubereitungen als wäßrige oder wäßrige-alkoholische (mit max. 15 Gew.-% Alkanol, bezogen auf das gesamte Mittel) Lösungen, Emulsionen, Gele, Cremes, Aerosole oder Lotionen formuliert werden.

Weiterhin können die erfindungsgemäßen Mittel alle in solchen Zubereitungen bekannten Wirk-, Zusatz- und Hilfsstoffe enthalten. In vielen Fällen enthalten die Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen. Anionische Tenside können dabei ganz besonders bevorzugt sein.

Erfindungsgemäß verwendbare Tenside sind beispielsweise:
- anionische Tenside wie insbesondere Seifen von Fettsäuren, Alkylsulfate, Alkylpolyglykolethersulfate und Ethercarbonsäuren mit 10 bis 18 C-Atomen in der Alkylgruppe und bis zu 12 Glykolethergruppen im Molekül, sowie Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- nichtionogene Tenside wie insbesondere Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe, C₁₂-C₂₂-Fettsäuremono- und -diester von Anlagerungsprodukten von 1 bis 30 Mol Ethylenoxid an Glycerin, C₈-C₂₂-Alkylmono- und -oligoglycoside und deren ethoxylierte Analoga sowie Anlagerungsprodukte von 5 bis 60 Mol Ethylenoxid an Rizinusöl und gehärtetes Rizinusöl,
   zwitterionische Tenside, insbesondere die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat,
- ampholytische Tenside wie N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe,
- kationische Tenside vom Typ der quartären Ammoniumverbindungen, z.B.Amrnoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, Behenyltrimethylammoniummethosulfat sowie die unter den INCI-Bezeichnungen Quaternium-27 und Quaternium-83 bekannten Imidazolium-Verbindungen. Ebenfalls als kationische Tenside einsetzbar sind Alkylamidoamine wie das unter der Bezeichnung Tegoamid® S 18 im Handel erhältliche Stearamidopropyldimethylamin und sogenannte Esterquats wie das unter der Bezeichnung Armocare® VGH-70 im Handel erhältliche N,N-Bis(2-Palmitoyloxyethyl)dimethylammoniumchlorid sowie die unter dem Warenzeichen Dehyquart® vertriebenen Produkte.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quatemisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert.Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobomylacrylat-Copolymere, Methylvinylether/Maleinsäureanhydrid-Copolymere und Acrylsäure/Ethylacrylat/N-tert.Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose, Maleinsäure und Milchsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein-, Mandelprotein und Weizenproteinhydrolysate, deren Kondensationsprodukte mit Fettsäuren sowie quaternisierte Proteinhydrolysate,
- Pflanzen- und Honigextrakte, wie insbesondere Extrakte aus Eichenrinden, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel,
- Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- Alkalisierungsmittel wie beispielsweise Ammoniak, Monoethanolamin, 2-Amino-2-methylpropanol und 2-Amino-2-methyl-propandiol-1,3
- weitere Substanzen zur Einstellung des pH-Wertes,
- Wirkstoffe wie Allantoin, Biotin, Bisabolol, Cholesterin sowie Pyrrolidoncarbonsäuren und deren Salze,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere,
- Perlglanzmittel wie Ethylenglykolmono- und -distearat sowie PEG-3-distearat,
- Komplexbildner wie EDTA, NTA, β-Alanindiessigsäure und Phosphonsäuren,
- Reduktionsmittel wie z.B. Thioglykolsäure und deren Derivate, Thiomilchsäure, Cysteamin, Thioäpfelsäure und α-Mercaptoethansulfonsäure,
- Oxidationsmittel wie Wasserstoffperoxid, Kaliumbromat und Natriumbromat sowie
- Antioxidantien.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe), sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Der pH-Wert der erfindungsgemäßen Mittel bei der Anwendung kann prinzipiell zwischen 2 - 11 liegen, wobei der Fachmann die bevorzugten pH-Bereiche für die unterschiedlichen Mittel kennt. Zur Einstellung des pH-Wertes kann praktisch jede für kosmetische Zwecke verwendbare Säure verwendet werden. Üblicherweise werden Genußsäuren verwendet. Unter Genußsäuren werden solche Säuren verstanden, die im Rahmen der üblichen Nahrungsaufnahme aufgenommen werden und positive Auswirkungen auf den menschlichen Organismus haben. Genußsäuren sind beispielsweise Essigsäure, Milchsäure, Weinsäure, Zitronensäure, Äpfelsäure, Ascorbinsäure und Gluconsäure. Im Rahmen der Erfindung ist die Verwendung von Zitronensäure und Milchsäure besonders bevorzugt.

Bezüglich weiterer Bestandteile sowie Mengenbereiche für die einzelnen Inhaltsstoffe wird auf die dem Fachmann bekannten Handbücher, z.B. K. Schrader, Grundlagen und Rezepturen der Kosmetika, 2. Auflage, Hüthig Buch Verlag, Heidelberg, 1989, verwiesen.

Selbstverständlich umfaßt die erfindungsgemäße Lehre auch solche Mittel, die mehrere, räumlich getrennte Zubereitungen A, von denen allerdings mindestens eine eine wäßrige Zubereitung darstellt, enthalten. Ebenso kann das erfindungsgemäße Mittel gewünschtenfalls auch mehrere, räumlich getrennte Zubereitungen B, die jeweils gemäß erfindungsgemäßer Lehre umhüllt sind, enthalten.

Gemäß einer speziellen Ausführungsform der erfindungsgemäßen Lehre kann es gewünscht sein, daß die Umhüllung der Zubereitung B so gestaltet ist, daß eine Vermischung der Zubereitung B mit einem wäßrigen Medium wie der Zubereitung A erst nach einer Latenzzeit von 20, insbesondere 30, Sekunden stattfindet. Dadurch wird für den Falle, daß beispielsweise bei einem Anwender mit feuchten oder nassen Händen, der eine Kapsel mit der Zubereitung B in ein Gefäß (z.B. eine Flasche) mit der Zubereitung A gibt, bereits Teile der Zubereitung während dieses Vorganges bereits an den Händen frei werden. Dies kann insbesondere in Hinblick auf lang haftende Parfümkomponenten eine unabdingbare Voraussetzung für die Praktikabilität des Verfahren und die Akzeptanz beim Anwender sein.

Ein weiterer Gegenstand der Erfindung ist die Verwendung eines Mittels nach einem der Ansprüche 1 bis 10 zur Behandlung menschlicher Haare.

Ein dritter Gegenstand der Erfindung ist schließlich ein Verfahren zur Behandlung menschlicher Haare, bei dem die Zubereitungen A und B eines Mittels nach einem der Ansprüche 1 bis 10 vereinigt werden und das erhaltene Mittel nach vollständiger Vermischung der Inhaltsstoffe auf das Haar aufgebracht und gegebenenfalls nach einer Einwirkzeit von 10 Sekunden bis 45 Minuten wieder abgespült wird.

## Patentansprüche

1. Mittel zur Behandlung keratinischer Fasern, bestehend aus mindestens einer wüLirigen Zubereitung A und mindestens einer davon räumlich getrennt vorliegenden Zubereitung B, die einen in der Zubereitung A nicht lagerstabilen Bestandteil, ausgewählt aus der Gruppe, die von Parfümölen sowie Vitaminen, Provitaminen und deren Derivaten gebildet wird, enthält, **gekennzeichnet durch** eine Umhüllung der Zubereitung B aus einem Material, das bei Zugabe der Zubereitung B zu einer Zubereitung A eine Vermischung der Komponenten beider Zubereitungen bei 38 °C innerhalb von 5 Minuten ermöglicht.

2. Mittel nach Anspruch 1, **dadurch gekennzeichnet, daß** die Umhüllung der Zubereitung B bei Zugabe der Zubereitung B zur einer Zubereitung A eine Vermischung der Komponenten beider Zubereitungen bei 20 °C innerhalb von 5 Minuten ermöglicht.

3. Mittel nach Anspruch 1 oder 2, **dadurch gekennzeichnet, daß** die Umhüllung der Zubereitung B eine Kapsel oder ein Säckchen ist.

4. Mittel nach Anspruch 3, **dadurch gekennzeichnet, daß** die Stärke der Umhüllung 10 bis 30 Mikrometer beträgt

5. Mittel nach einem der Ansprüche 1 bis 4, **dadurch gekennzeichnet, daß** die Umhüllung aus einem einheitlichen Material besteht..

6. Mittel nach einem der Ansprüche 1 bis 5, daß die Umhüllung zumindest teilweise aus Polyvinylalkohol besteht.

7. Mittel nach einem der Ansprüche 1 bis 6, **dadurch gekennzeichnet, daß** es sich um ein Mittel zum Färben, Blondieren oder dauerhaften Verformen von menschlichen Haaren handelt.

8. Verwendung eines Mittels nach einem der Ansprüche 1 bis 7 zur Behandlung menschlicher Haare.

9. Verfahren zur Behandlung menschlicher Haare, **dadurch gekennzeichnet, daß** die Zubereitungen A und B eines Mittels nach einem der Ansprüche 1 bis 7 vereinigt werden und das erhaltene Mittel nach vollständiger Vermischung der Inhaltsstoffe auf das Haar aufgebracht und gegebenenfalls nach einer Einwirkzeit von 10 Sekunden bis 45 Minuten wieder abgespült wird.

## Claims

1. A preparation for treating keratin fibers, comprising at least one aqueous preparation A and, spatially separate therefrom, at least one preparation B, which comprises a constituent which is not storage-stable in the preparation A, and is selected from the group comprising perfume oils, and vitamins, provitamins and derivatives thereof, the preparation B being contained in an envelope of material which permits the components of the two preparations to mix at 38 °C within 5 minutes when preparation B is added to preparation A.

2. The preparation as claimed in claim 1, wherein the envelope of preparation B permits the components of the two preparations to mix at 20°C within 5 minutes when preparation B is added to preparation A.

3. The preparation as claimed in claim 1 or 2, wherein the envelope of preparation B is contained in a capsule of a pouch.

4. The preparation as claimed in claim 3, wherein the capsule or pouch is 10 to 30 micrometers in size.

5. The preparation as claimed in any of claims 1 to 4, wherein the envelope is a uniform material.

6. The preparation as claimed in any of claims 1 to 5, wherein the envelope consists at least partially of polyvinyl alcohol.

7. The preparation as claimed in any of claims 1 to 6, which is an agent for the coloring, bleaching or permanent shaping of human hair.

8. The use of a preparation as claimed in any of claims 1 to 7 for the treatment of human hair.

9. A method of treating human hair, which comprises combining the preparations A and B as claimed in any of claims 1 to 7 and, following complete mixing of the ingredients, applying the resulting agent to the hair, and optionally after a contact time of from 10 seconds to 45 minutes rinsing it off again.

## Revendications

1. Agent pour le traitement des fibres kératiniques consistant en au moins une préparation aqueuse A et au moins une préparation B qui se présente séparée dans l'espace et qui contient un constituant non stable au stockage dans la préparation A, choisi dans le groupe formé par des essences de parfum, ainsi que par des vitamines, des provitamines et par leurs dérivés,
**caractérisé par**
un enrobage de la préparation B à base d'un matériau qui rend possible par addition de la préparation B à une préparation A, un mélange des composants des deux préparations à 38°C en l'espace de 5 minutes.

2. Agent selon la revendication 1,
**caractérisé en ce que**
l'enrobage de la préparation B rend possible par addition de la préparation B à une préparation A, un mélange des composants des deux préparations à 20°C en l'espace de 5 minutes.

3. Agent selon la revendication 1 ou la revendication 2,
**caractérisé en ce que**
l'enrobage de la préparation B est une capsule ou un petit sac.

4. Agent selon la revendication 3,
**caractérisé en ce que**
l'épaisseur de l'enrobage s'élève à 10 à 30 micromètres.

5. Agent selon l'une des revendications 1 à 4,
**caractérisé en ce que**
l'enrobage consiste en un matériau homogène.

6. Agent selon l'une des revendications 1 à 5,
**caractérisé en ce que**
l'enrobage consiste au moins en partie en de l'alcool polyvinylique.

7. Agent selon l'une des revendications 1 à 6,
**caractérisé en ce que**
il s'agit d'un agent pour teinture, blondissage ou déformation permanente des cheveux humains.

8. Utilisation d'un agent selon l'une des revendications 1 à 7, pour le traitement des cheveux humains.

9. Procédé de traitement des cheveux humains,
**caractérisé en ce que**
les préparations A et B d'un agent selon l'une des revendications 1 à 7 sont réunies, et l'agent obtenu est appliqué sur les cheveux après mélange complet des ingrédients, et, le cas échéant après un temps d'action allant de 10 secondes à 45 minutes, est à nouveau éliminé par rinçage.
